# EUROPEAN PATENT APPLICATION

(11) **EP 2 907 447 A1**
(43) Date of publication of application: **19.08.2015**
(21) Application number: 14175260.0
(22) Date of filing: 01.07.2014
(51) Int. Cl.: A61B 5/024, A61B 5/00

(54) **Heartbeat detecting bracelet**

(30) Priority: 13.02.2014 TW 103202527
(71) Applicant: Cheng Uei Precision Industry Co., Ltd., New Taipei City 236 (TW)
(72) Inventor: Lee, James Cheng, 236 New Taipei City (TW)
(74) Representative: Gee, Steven William

(57) **Abstract**

A heartbeat detecting bracelet includes a base body, a sensor, a processing module and a band-like body. The base body is equipped with a first electrode slice located in an inner surface of the base body for being attached to the skin of the human body, and a second electrode slice located in any one surface of the base body except the inner surface for being touched in use. The sensor is disposed to the base body and capable of being touched or approached during the second electrode slice is touched. The processing module is disposed in the base body and coupled with the first electrode slice, the second electrode slice and the sensor for powering on or off the heartbeat detecting bracelet according to signals from the sensor. The band-like body is combined with the base body for wearing the heartbeat detecting bracelet to the human body.

## Description

### CROSS-REFERENCE TO RELATED APPLICATION

The present application is based on, and claims priority form, Taiwan Patent Application No. 103202527, filed Feb. 13, 2014, the disclosure of which is hereby incorporated by reference herein in its entirety.

### BACKGROUND OF THE INVENTION

### 1. Field of the Invention

The present invention relates to a heartbeat detecting bracelet, and more particularly to a user-friendly heartbeat detecting bracelet.

### 2. The Related Art

Electrocardiography (ECG) is the recording of the electrical activity of the heart. Traditionally this is in the form of a transthoracic interpretation of the electrical activity of the heart over a period of time, as detected by electrodes attached to the surface of the skin and recorded or displayed by a device external to the body. The recording produced by this noninvasive procedure is termed an electrocardiogram through which the individual physiological conditions are gotten and personal health can be paid attention as early as possible. So the ECG is widely used in the sports and medical field.

The ECG picks up electrical impulses generated by the polarization and depolarization of cardiac tissue and translates into a waveform. The waveform is then used to measure the rate and regularity of heartbeats, as well as the size and position of the chambers, the presence of any damage to the heart, and the effects of drugs or devices used to regulate the heart, such as a pacemaker. Because each person's physiological organization has different impact on ECG signals, a normal variation is generated to get different fixed details for each person in the electrocardiogram. The biological characteristics of the different fixed details have been successfully applied in the field of individual identification, such as unlocking doors, unlocking phones and unlocking software. Therefore, a heartbeat detecting device used with the ECG has a wide application area extending beyond the sports and medical field to people's daily lives.

A heartbeat detecting bracelet has been a daily tool of people's lives on account of its portability. However, at present, the known heartbeat detecting bracelet often consumes more power even when not in use. In order to save power, the heartbeat detecting bracelet needs to be manually powered off when it is idle. As a result, it is inconvenient for users to firstly turn on the power switch before using the heartbeat detecting bracelet. For overcoming the foregoing problem, an improved heartbeat detecting bracelet is required.

### SUMMARY OF THE INVENTION

An object of the present invention is to provide a heartbeat detecting bracelet which includes a base body, a sensor, a processing module and a band-like body. The base body is equipped with a first electrode slice and a second electrode slice. The first electrode slice is located in an inner surface of the base body for being attached to the skin of the human body. The second electrode slice is located in any one surface of the base body except the inner surface for being touched in use. The sensor is disposed to the base body and capable of being touched or approached during the second electrode slice is touched. The processing module is disposed in the base body and coupled with the first electrode slice, the second electrode slice and the sensor for powering on or off the heartbeat detecting bracelet according to signals from the sensor. The band-like body is combined with the base body for wearing the heartbeat detecting bracelet to the human body.

As described above, the heartbeat detecting bracelet of the present invention can be automatically powered on after the user touches the second electrode slice to complete the measuring posture, and further can be automatically powered off after the second electrode slice is free to complete the measurement. So it can effectively save power, and moreover is convenient for using and has more practicability in people's daily lives.

### BRIEF DESCRIPTION OF THE DRAWINGS

The present invention will be apparent to those skilled in the art by reading the following description thereof, with reference to the attached drawings, in which:
FIG. 1 is a circuit block diagram of a heartbeat detecting bracelet according to the present invention;
FIG. 2 is an assembled perspective view of the heartbeat detecting bracelet according to the present invention;
FIG. 3 is a partial enlarged view of the heartbeat detecting bracelet according to the first embodiment of the present invention, which shows the first way to set a sensor and a second electrode slice;
FIG. 4 is a partial enlarged view of the heartbeat detecting bracelet according to the second embodiment of the present invention, which shows the second way to set the sensor and the second electrode slice;
FIG. 5 is a partial enlarged view of the heartbeat detecting bracelet according to the third embodiment of the present invention, which shows the third way to set the sensor and the second electrode slice;
FIG. 6 is a partly exploded perspective view of the heartbeat detecting bracelet of FIG. 2; and
FIG. 7 is a cross-sectional view of the heartbeat detecting bracelet except a band-like body of FIG. 6.

### DETAILED DESCRIPTION OF THE EMBODIMENT

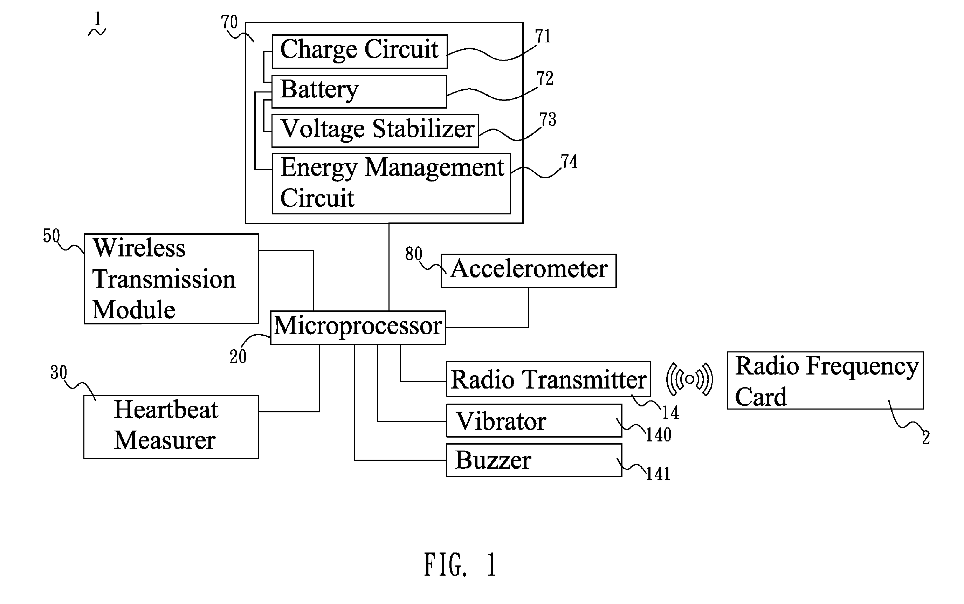

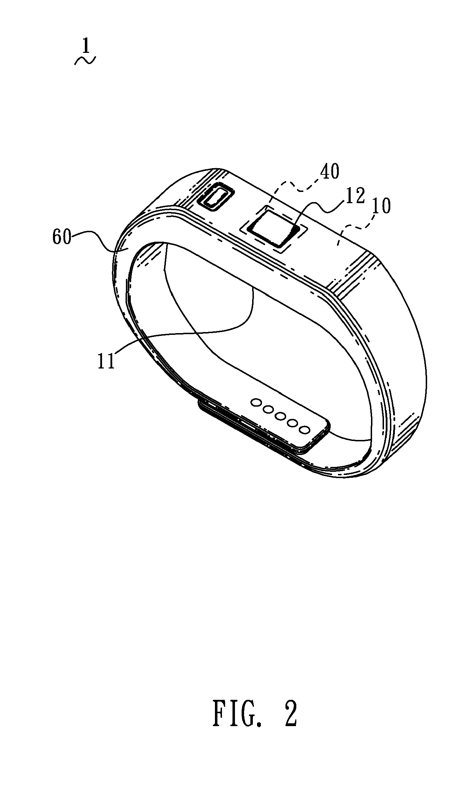

Referring to FIG. 1 and FIG. 2, a heartbeat detecting bracelet 1 according to the present invention includes a base body 10, a microprocessor 20, a heartbeat measurer 30, a sensor 40, a wireless transmission module 50 and a band-like body 60. The microprocessor 20, the heartbeat measurer 30 and the wireless transmission module 50 are disposed in the base body 10 and cooperatively form a processing module, wherein the microprocessor 20 is coupled with the heartbeat measurer 30 and the wireless transmission module 50. The sensor 40 is disposed to the base body 10 and coupled with the microprocessor 20. The band-like body 60 is combined with the base body 10 for fastening the heartbeat detecting bracelet 1 to the human body.

The base body 10 is equipped with a first electrode slice 11 and a second electrode slice 12 of which both are coupled with the heartbeat measurer 30. In this invention, the first electrode slice 11 is located in an inner surface of the base body 10, and the second electrode slice 12 is located in any one surface of the base body 10 except the inner surface. When the heartbeat detecting bracelet 1 is worn to one wrist of the human body by virtue of the band-like body 60, the first electrode slice 11 is tightly attached to the skin of the wrist. Then in use, the other hand of the human body touches the second electrode slice 12, so that a circuit loop is formed through the heartbeat detecting bracelet 1, the left and right hands and the heart of the human body for the heartbeat measurer 30 measuring ECG signals.

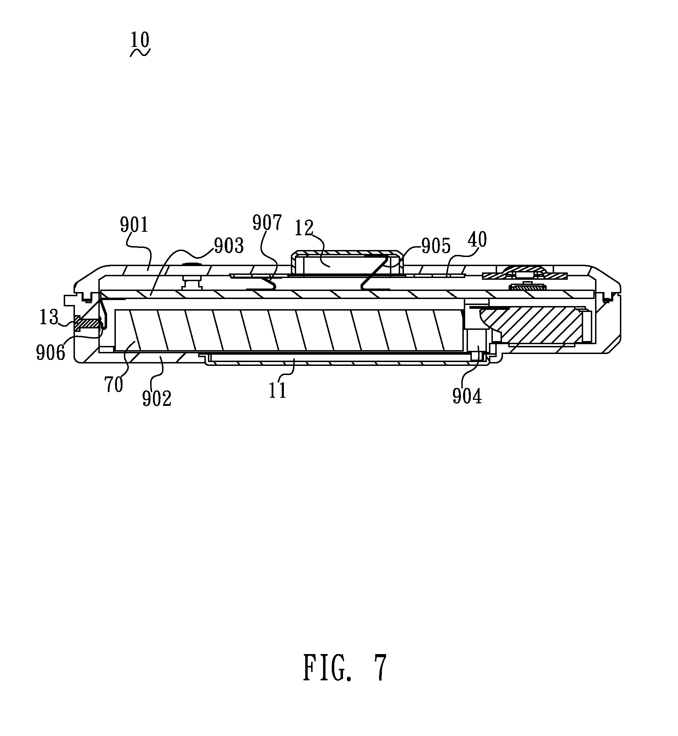

The heartbeat detecting bracelet 1 further includes a power module 70 disposed in the base body 10 and coupled with the microprocessor 20. The base body 10 is further equipped with a plurality of electrical terminals 13 (referring to FIG. 7) coupled with the power module 70 and the microprocessor 20 and exposed outside the base body 10. The power module 70 includes a charge circuit 71, a battery 72, a voltage stabilizer 73 and an energy management circuit 74. The positive pole and the negative pole of the charge circuit 71 are connected with one each electrical terminal 13 respectively. The charge circuit 71 is coupled with the battery 72 and the microprocessor 20, the battery 72 is coupled with the voltage stabilizer 73 and the energy management circuit 74, and both the energy management circuit 74 and the voltage stabilizer 73 are coupled with the microprocessor 20.

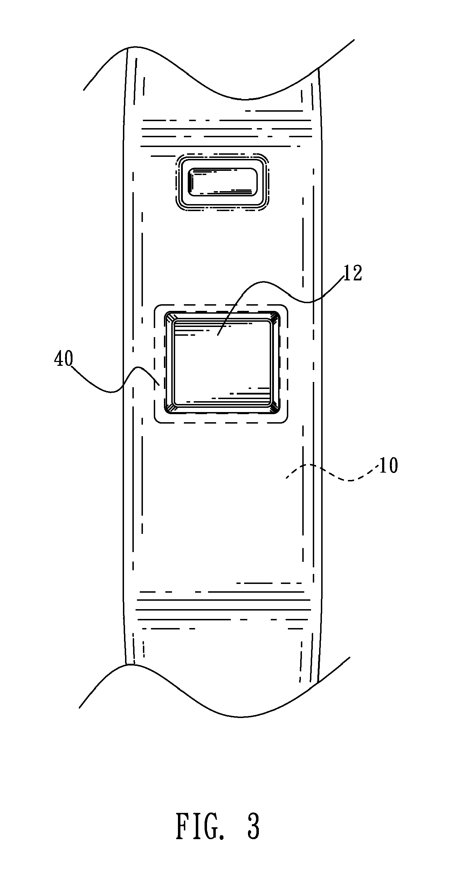

Referring to FIG. 3, it shows a first embodiment of the present invention. In the first embodiment, the sensor 40 is located inside the base body 10 and stays close to the second electrode slice 12 for sensing whether the second electrode slice 12 is touched. When the other hand of the human body touches the second electrode slice 12, because it is close to the sensor 40 at the same time, the microprocessor 20 receives signals sensed by the sensor 40 to realize the circuit loop through the heartbeat measurer 30 and simultaneously power on the heartbeat measurer 30.

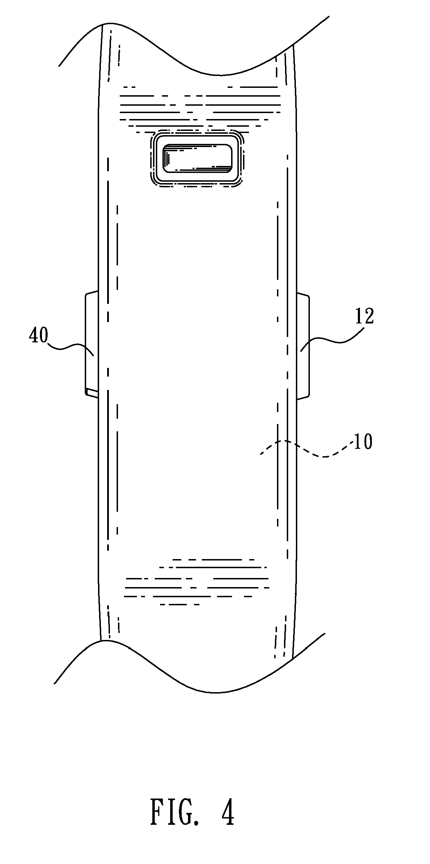

Referring to FIG. 4, it shows a second embodiment of the present invention. In the second embodiment, the second electrode slice 12 and the sensor 40 are disposed in two opposite lateral sides of the base body 10 and exposed outside the base body 10. In use, any two fingers of the other hand simultaneously touch and press the second electrode slice 12 and the sensor 40 on the base of the base body 10. This action can realize the circuit loop through the heartbeat detecting bracelet 1, the left and right hands and the heart of the human body, and directly power on the heartbeat measurer 30 for the convenience of using. Furthermore, the contact area of the finger and the second electrode slice 12 can be increased by apply more force on the base body 10.

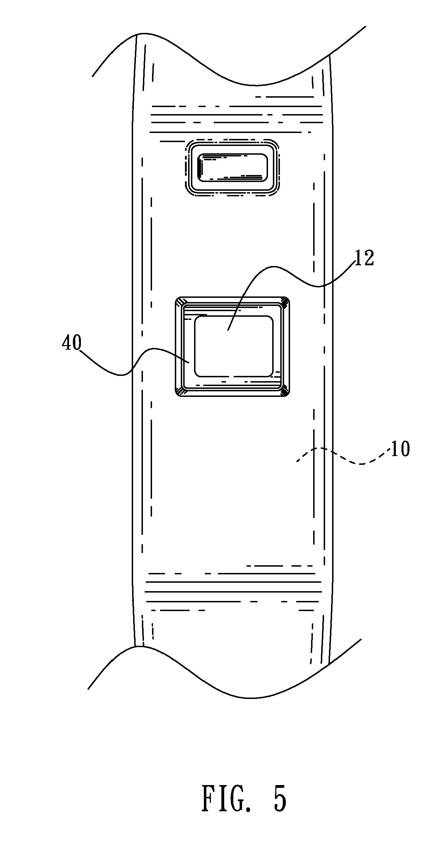

Referring to FIG. 5, it shows a third embodiment of the present invention. In the third embodiment, the sensor 40 is disposed in the same surface of the base body 10 as the second electrode slice 12 and adjacent to the second electrode slice 12. The second electrode slice 12 and the sensor 40 are exposed outside the base body 10. When the finger touches the second electrode slice 12, the sensor 40 will be simultaneously touched by the finger to power on the heartbeat measurer 30.

In this invention, the sensor 40 is preferably a capacitive sensor with low-power and quick-acting. The microprocessor 20 can automatically power off the heartbeat measurer 30 according to the signals from the sensor 40 when the sensor 40 is in a state of no touch, so the heartbeat detecting bracelet 1 can effectively save power. But the sensor 40 is not limited to the capacitive sensor, as long as it can generate signals by touching or induction.

Powering off the heartbeat measurer 30 is operated by the microprocessor 20 by means of judging whether the circuit loop is formed through the human body or according to the signals from the sensor 40.

After completion of the measurement, the microprocessor 20 gains measured data from the heartbeat measurer 30 and then utilizes the wireless transmission module 50 to transmit the measured data to an external electronic device, such as a wrist watch, a mobile phone and a computer, so as to display the measured data on the electronic device or make a biological signature for the default action in the electronic device. The wireless transmission module 50 is a signal transmitter capable of transmitting radio frequency identification signals, Bluetooth signals, Z-wave signals, wireless USB signals, or composite signals from the foregoing ones. But the wireless transmission module 50 is not limited to the foregoing signal transmitter, as long as it can wirelessly transmit the measured data to the electronic device.

Referring to FIG. 1, because the heartbeat detecting bracelet 1 is suitable for long time wearing on account of the basic function thereof having extended all over in people's everyday life, in order to increase conveniences of the heartbeat detecting bracelet 1, the base body 10 is further equipped with a radio transmitter 14, a vibrator 140 and a buzzer 141 which are coupled with the microprocessor 20 respectively. The radio transmitter 14 is used to sense a radio frequency card 2 which is installed in a specified item (not shown) by the user. If the specified item is at a distance from the heartbeat detecting bracelet 1, the microprocessor 20 controls the vibrator 140 or the buzzer 141 to give an alarm so as to prevent the user's belongings from being lost.

Referring to FIG. 1 again, further, the base body 10 is equipped with an accelerometer 80 coupled with the microprocessor 20 so as to make the electronic device receiving the measured data from the heartbeat detecting bracelet 1 capable of processing the activity information of the measured user.

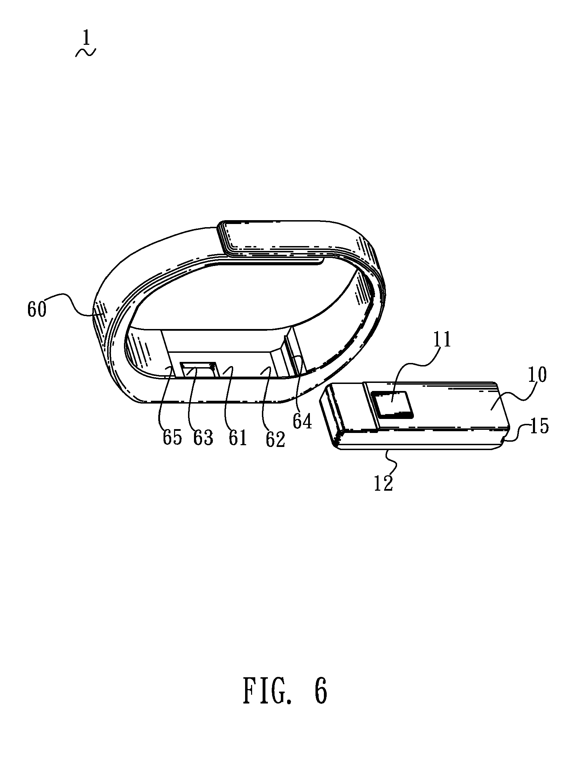

Referring to FIG. 6, for the convenience of charging the battery 72 of the power module 70, the band-like body 60 defines a holding cavity 61 where the base body 10 is detachably assembled. The band-like body 60 is made from plastics or ebonite with stress deformation feature. Two opposite sidewalls of the holding cavity 61 are opened with a first window 62 corresponding to the first electrode slice 11 and a second window 63 corresponding to the second electrode slice 12. At least one locking latch 64 is protruded on the inner side of the holding cavity 61. Accordingly, the lateral side of the base body 10 defines at least one locking groove 15 corresponding to the locking latch 64 of the band-like body 60. One side edge of the first window 62 defines a blocking slice 65 projecting in the first window 62.

In assembly, the base body 10 is positioned in the holding cavity 61 of the band-like body 60 through the first window 62 until the locking latch 64 is buckled in the locking groove 15 by virtue of elastic deformation. The blocking slice 65 resists against the inner surface of the base body 10 in which the first electrode slice 11 is located so as to block the base body 10 from falling out of the holding cavity 61. The first electrode slice 11 is exposed outside through the first window 62 and the second electrode slice 12 is exposed outside through the second window 63.

Referring to FIG. 7, in a concrete embodiment, the base body 10 includes an outer shell 901 and an inner shell 902 mating and covered with each other between which a circuit board 903 is disposed. The microprocessor 20, the heartbeat measurer 30, the wireless transmission module 50 and the power module 70 are located on the circuit board 903. The first electrode slice 11 is embedded through the inner shell 902 and electrically connected to the circuit board 903 by a first resilient terminal 904, and the second electrode slice 12 is embedded through the outer shell 901 and electrically connected to the circuit board 903 by a second resilient terminal 905. The electrical terminals 13 are embedded through one side wall of the inner shell 902. The circuit board 903 is further provided with a plurality of third resilient terminals 906 electrically abutting against the electrical terminals 13 to form a circuit of the circuit board 903 and the electrical terminals 13.

The sensor 40 is a capacitive sensor and disposed on the inner side of the outer shell 901 around the second electrode slice 12. The sensor 40 is electrically connected to the circuit board 903 by a fourth resilient terminal 907, so that the microprocessor 20 can receive the signals from the sensor 40 when the second electrode slice 12 is touched by the finger.

The first resilient terminal 904, the second resilient terminal 905, the third resilient terminals 906 and the fourth resilient terminal 907 are metal resilient elements, such as flexible strips or pogo pins, so that the circuit board 903 can be easily and directly detached from the outer shell 901 and the inner shell 902 but without being limited by the elements.

As described above, the heartbeat detecting bracelet 1 of the present invention can automatically power on the heartbeat measurer 30 after the user completes the measuring posture, and further can automatically power off the heartbeat measurer 30 after the measurement is completed. So it can effectively save power, and moreover is convenient for using and has more practicability in people's daily lives.

The foregoing description of the present invention has been presented for purposes of illustration and description. It is not intended to be exhaustive or to limit the invention to the precise form disclosed, and obviously many modifications and variations are possible in light of the above teaching. Such modifications and variations that may be apparent to those skilled in the art are intended to be included within the scope of this invention as defined by the accompanying claims.

## Claims

1. A heartbeat detecting bracelet, comprising:
a base body equipped with a first electrode slice and a second electrode slice, the first electrode slice being located in an inner surface of the base body for being attached to the skin of the human body, the second electrode slice being located in any one surface of the base body except the inner surface for being touched in use;
a sensor disposed to the base body and capable of being touched or approached during the second electrode slice is touched;
a processing module disposed in the base body and coupled with the first electrode slice, the second electrode slice and the sensor for powering on or off the heartbeat detecting bracelet according to signals from the sensor; and
a band-like body combined with the base body for wearing the heartbeat detecting bracelet to the human body.

2. The heartbeat detecting bracelet as claimed in claim 1, wherein the second electrode slice and the sensor are disposed in two opposite lateral sides of the base body and exposed outside the base body for being touched and pressed simultaneously in use.

3. The heartbeat detecting bracelet as claimed in claim 1, wherein the sensor is disposed in the same surface of the base body as the second electrode slice and adjacent to the second electrode slice, the sensor and the second electrode slice are exposed outside the base body for being simultaneously touched.

4. The heartbeat detecting bracelet as claimed in claim 1, wherein the sensor is located inside the base body and stays close to the second electrode slice for sensing whether the second electrode slice is touched.

5. The heartbeat detecting bracelet as claimed in claim 4, wherein the band-like body defines a holding cavity where the base body is detachably assembled, two opposite sidewalls of the holding cavity are opened with a first window corresponding to the first electrode slice and a second window corresponding to the second electrode slice, at least one locking latch is protruded on the inner side of the holding cavity, the lateral side of the base body defines at least one locking groove corresponding to the locking latch of the band-like body, the base body is positioned in the holding cavity of the band-like body through the first window until the locking latch is buckled in the locking groove, the first electrode slice is exposed outside through the first window and the second electrode slice is exposed outside through the second window.

6. The heartbeat detecting bracelet as claimed in claim 5, wherein one side edge of the first window defines a blocking slice projecting in the first window and blocking against the inner surface of the base body in which the first electrode slice is located.

7. The heartbeat detecting bracelet as claimed in claim 5, wherein the base body includes an outer shell and an inner shell mating and covered with each other between which a circuit board is disposed, the processing module is located on the circuit board, the first electrode slice is embedded through the inner shell and electrically connected to the circuit board by a first resilient terminal, and the second electrode slice is embedded through the outer shell and electrically connected to the circuit board by a second resilient terminal.

8. The heartbeat detecting bracelet as claimed in claim 7, further comprising a power module disposed in the base body and coupled with the processing module, the base body is further equipped with a plurality of electrical terminals coupled with the power module and the processing module and exposed outside the base body, the power module includes a charge circuit, a battery, a voltage stabilizer and an energy management circuit, the positive pole and the negative pole of the charge circuit are connected with one each electrical terminal respectively, the charge circuit is coupled with the battery and the processing module, the battery is coupled with the voltage stabilizer and the energy management circuit, and both the energy management circuit and the voltage stabilizer are coupled with the processing module.

9. The heartbeat detecting bracelet as claimed in claim 8, wherein the electrical terminals are embedded through one side wall of the inner shell, the circuit board is further provided with a plurality of third resilient terminals electrically abutting against the electrical terminals to form a circuit of the circuit board and the electrical terminals.

10. The heartbeat detecting bracelet as claimed in claim 7, wherein the sensor is disposed around the second electrode slice, and the sensor is electrically connected to the circuit board by a fourth resilient terminal.

11. The heartbeat detecting bracelet as claimed in claim 4, wherein the base body is further equipped with a radio transmitter, a vibrator and a buzzer which are respectively coupled with the processing module, the radio transmitter is used to sense a radio frequency card.

12. The heartbeat detecting bracelet as claimed in claim 4, wherein the base body is further equipped with an accelerometer coupled with the processing module.
